# EUROPEAN PATENT APPLICATION

(11) **EP 4 614 507 A1**
(43) Date of publication of application: **10.09.2025**
(21) Application number: 24162161.4
(22) Date of filing: 07.03.2024
(51) Int. Cl.: G16H 20/70, G16H 50/20, G16H 10/60, G16H 80/00

(54) **IN-HOME-APPARATUS AND REMOTE PATIENT MONITORING SYSTEM IN PARTICULAR FOR MONITORING A PATIENT´S MENTAL STATUS**

(71) Applicant: Deutsche Telekom AG, 53113 Bonn (DE)
(72) Inventor: KIM, Ju Hoon, 14947 Nuthe-Urstromtal (DE)
(74) Representative: Blumbach · Zinngrebe Patentanwälte PartG mbB

(57) **Abstract**

The present invention relates to an in-home apparatus (200) and to a remote patient monitoring system (10). The in-home apparatus (200) comprises a device (210) having a light reflecting area, a camera (204), a first communication interface (202) configured to connect the apparatus (200) to a telecommunication network (800) for communicating with an external computing system (300), a display (207) having a touch screen functionality, a microphone (205) and a speaker (206, a processing unit (211) configured
a) to cause the apparatus (200) to collect patient related data including image data captured by the camera (204), and/or vital sign data provided by at least one vital sign sensor device (900, 910) configured to detect vital signs of the patient and to generate vital sign data, and/or communication data input by the patient via the display (207) and/or the microphone (205), and
b) to cause the apparatus (200) to deliver the collected patient related data to the first communication interface (202) for transmitting them to the external computing system (300).

## Description

### Technical Field of the Invention

The present invention relates to an in-home apparatus in particular for use in a remote patient monitoring system and a remote patient monitoring system in particular for monitoring a patient's mental status.

### Background of the Invention

Technically based health care systems for remote monitoring patients living at home are known. For example, German utility model DE 20 2021 105 877 U1 discloses a system for remote patient monitoring for Covid 19 infected persons. In the European patent EP 1 805 678 B1 an in-home remote monitor with smart repeater, memory and emergency event management is disclosed.

It is an object of the present invention to provide an in-home apparatus and a remote patient monitoring system including such an in-home apparatus, which enable a patient to interact and/or to have a conversation with the in-home apparatus and the remote patient monitoring system, respectively, in order to monitor a patient's mental status and in particular to monitor a patient suffering from depressive disorders and to provide treatment measures for the patient, if required.

### Brief Summary of the Invention

The technical problem is solved by independent claims 1, 9 and 10. Preferred embodiments are set forth in the dependent claims.

According to a first aspect, an in-home apparatus in particular for use in a remote patient monitoring system is provided, which is preferably configured to collect and transmit patient related data to an external computing system. The in-home apparatus preferably comprises the following features:
a device having a light reflecting area configured to show a mirrored image of a patient,
a camera configured to capture image data of the patient,
a first communication interface configured to connect the in-home apparatus to a telecommunication network for communicating with an external computing system connectable to the telecommunication network,
a display having a touch screen functionality, a microphone and a speaker,
a processing unit configured
   (a) to cause the in-home apparatus to collect patient related data including the image data and/or vital sign data provided by at least one sensor device configured to detect vital signs of the patient and to generate vital sign data and/or communication data input by the patient via the display and/or the microphone,
   (b) to cause the in-home apparatus to deliver the collected patient related data to the first communication interface for transmitting them to the external computing system.

In particular, communication data represents answer information input by the patient via the display and/or the microphone in response to treatment related information output via the speaker and/or the display. The treatment related information includes for example at least one instruction and/or at least one question for the patient and/or multimedia data.

Such an in-home apparatus can be considered as a mirror-like smart apparatus.

It is to be noted, that the first communication interface can be configured to directly connect the in-home-apparatus to the telecommunication network. Alternatively, the first communication interface can be implemented according to a standardized WiFi or Ethernet communication protocol to enable communication with an in-home router.

It is further to be noted, that the processing unit is preferably implemented as a microprocessor or a microcontroller.

In a cost effective manner, the device having an optical reflecting area is preferably implemented as a conventional mirror.

Preferably, the device having an optical reflecting area and the display can be implemented integrally such that the optical reflecting area is configured to operate as a mirror and/or a display, wherein the optical reflecting area has a touch screen functionality. Such a device may be called a touch-enabled mirror-display device.

Such a smart mirror touchscreen can be found for example disclosed under the URL https://smartbuilds.io/smart-mirror-touchscreen-raspberry-pi/.

Smart mirrors are for example described in US 2018/0343418 A1 and US 10.142.592 B1.

The display can be implemented as a liquid-crystal display (LCD).

The display and/or the microphone and/or the speaker are preferably arranged at the in-home apparatus, for example on a front side of the in-home apparatus. Alternatively, they can be communicatively connected as external components via a wireless or wired link to the in-home apparatus.

Preferably, the at least one vital sign sensor device is an external sensor device and wherein the in-home apparatus further comprises at least one second communication interface configured to be communicatively coupled with the external vital sign sensor device. Preferably, the at least one second communication interface is implemented according to a short-range wireless communication protocol, e.g. Bluetooth, and wherein the at least one external sensor device is a wearable sensor device or an implantable sensor device with a respective communication interface.

Alternatively, the at least one vital sign sensor device may be an integral vital sign sensor device of the in-home apparatus.

It is be noted, that a plurality of vital sign sensor devices can be provided. In this case, at least one of the plurality of vital sign sensor devices can be realized as an integral vital sign sensor device of the in-home apparatus and at least one further of the plurality of vital sign sensor device can be an external vital sign sensor device.

Preferably, the vital sign data represents the body temperature, the blood pressure, the heart rate or the respirating rate of the patient.

Preferably, the processing unit is configured to cause the in-home apparatus
- to execute a speech-to-text conversion on speech signals input by the patient via the microphone and/or
- to execute a text-to-speech conversion on data representing text received from the external computing system and to output the corresponding speech signals via the speaker.

Preferably the processing unit of the in-home apparatus may be further configured to cause the in-home apparatus to execute or initiate execution of an authentication procedure between the in-home apparatus and the external computing system, and/or to decrypt encrypted data received from the external computing system and to encrypt patient related data destined for the external computing system and/or to grant only an authorized patient access to the in-home apparatus. An authorized patient can be detected by the in-home apparatus by using, for example, a fingerprint sensor or preferably by processing face data captured by the camera. In this case, a software including a face recognition program can be stored in the in-home apparatus. Therefore, the processing unit can be configured to execute the face recognition program using face data captured by the camera and to cause the in-home apparatus to grant access if an authorized patient is detected.

In order to extend the functionality, the in-home apparatus may further comprise a storage device configured to store software including a facial expression recognition program, wherein the processing unit is configured to obtain when executing the facial expression recognition program face data from the image data, the face data representing facial expressions of the patient and to cause the apparatus to deliver the face data as patient related data to the first communication interface for transmitting them to the external computing system.

According to a second aspect a remote patient monitoring system in particular for monitoring a patient's mental status is provided, which preferably comprises the following features:
an in-home apparatus of claim 9, the in-home apparatus located at a patient's home,
a telecommunication network,
a first external computing system, the first external computing system and the in-home apparatus being configured to communicate with each other via the telecommunication network, wherein
the first external computing system is configured to receive from the in-home apparatus first patient related data including vital sign data of the patient and/or face data representing facial expressions of the patient and/or communication data input by the patient via the microphone and/or the display, wherein the first external computing system comprises:
   - a communication interface configured to connect the external computing system to the telecommunication network,
   - a storage device, and
   - a processing unit configured
to analyze the first patient related data received from the in-home apparatus by executing an AI-based algorithm to determine the patient's mental status, to provide in dependence of the determined patient's mental status treatment related information including at least one instruction and/or at least one question for the patient, and
to transmit the treatment related information to the in-home apparatus, wherein the in-home apparatus is configured to output the treatment related information received from the first external computing system via the display and/or the speaker.

It is to be note, that the telecommunication network is preferably a wide area network which may comprise the Internet and in dependence of the implementation wireless and/or wired access networks over which the in-home apparatus and the external computing system can be connected to each other. Preferably, the telecommunication network is an IP-based telecommunication network.

It is to be noted, that the AI-based algorithm uses in particular a machine learning algorithm, wherein preferably vital sign data of the patient and/or face data representing facial expressions of the patient and/or communication data input by the patient via the microphone and/or the display can be use an input data of the AI-based algorithm.

According to a third aspect a remote patient monitoring system in particular for monitoring a patient's mental status is provided, which preferably comprises the following features:
an in-home apparatus of any one of the claims 1 to 7, the in-home apparatus located at a patient's home,
a telecommunication network,
a first external computing system, the first external computing system and the in-home apparatus being configured to communicate with each other via the telecommunication network, wherein
the first external computing system is configured to receive from the in-home apparatus first patient related data including image data of the patient and/or vital sign data of the patient and/or communication data input by the patient via the microphone and/or the display, wherein
the first external computing system comprises:
   a communication interface configured to connect the first external computing system to the telecommunication network,
   a storage device configured to store software including a facial expression recognition program, and
   a processing unit configured to cause the first external computing system
      - to obtain when executing the facial expression recognition program face data from the image data received from the in-home apparatus, the face data representing facial expressions of the patient,
      - to analyze the obtained face data and/or the vital sign data and/or the communication data received from the in-home apparatus by executing an AI-based algorithm to determine the patient's mental status,
   to provide in dependence of the determined patient's mental status treatment related information including at least one instruction and/or at least one question for the patient, and
   to transmit the treatment related information to the in-home apparatus, wherein the in-home apparatus is configured to output the treatment related information received from the first external computing system via the display and/or the speaker.

It is to be noted, that communication data preferably representing answer information input by the patient in response to treatment related information previously received from the first external computing system or in response to initial questions provided by the in-home apparatus itself.

It is to be noted, that the step of providing treatment related information in dependence of the determined patient's mental status can preferably include the step of selecting a predetermined treatment related information from a plurality of predetermined treatment related information stored in the first external computing system, or, more sophisticated, the step of generating a treatment related information by selecting treatment elements from a pool of predetermined treatment elements stored in the first external computing system. A treatment element is for example a high-level action including multimedia content like a plurality of pictures, a video, a piece of music and/or a sports exercise, a text, a predetermined question or a predetermined instruction. An exemplary predetermined related information for a specific patient may comprise at least one instruction, e.g. "Select two pictures", "Read the text aloud", "Watch a sport video and do the shown exercises", "Listen to the music", "Go to the location A and count the chairs in that area", "Watch a video A", "Draw a picture", etc., and/or at least one question and, if applicable, multimedia content, e.g. visual and/or textual and/or acoustical information. Visual information can include a plurality of pictures, a video. Textual information can include an article or an excerpt of a book. Acoustical information can be a spoken text or a song.

In order to enable a more personal conversation with the patient, either
a) the remote patient monitoring system may preferably comprises a second external computing system configured to execute a generative AI-based algorithm, wherein the processing unit of the first external computing system is configured - to cause the first external computing system to prompt the second external computing system to execute the generative AI-based algorithm to generate a human-like conversation sentence using the at least one question and/or the at least one instruction of the treatment related information provided by the first external computing system, wherein the second external computing system is configured to transmit the human-like conversation sentence to the first external computing system, or without using a second external computing system,
b) the processing unit of the first external computing system is configured to cause the first external computing system to execute a generative AI-based algorithm stored on the storage device to generate a human-like conversation sentence with respect to the at least one predetermined instruction provided by the first external computing system, wherein
   the processing unit of the first external computing system is configured to cause the first external computing system to transmit the human-like conversation sentence to the in-home-apparatus, wherein
   the processing unit of the in-home apparatus is configured to cause the in-home-apparatus to output the human-like conversation sentence via the display and/or the speaker.

The generative AI-based algorithm can use a Large Language Model (LLM) like a generative pre-trained transformer (GPT) used for example in ChatGPT.

In order to improve the interaction and/or conversation between the patient and the remote patient monitoring system and the in-home apparatus, respectively, the treatment related information provided by the first external computing system in dependence of the determined patient's mental status further includes multimedia information like visual, and/or textual and/or acoustical information in particular associated with the at least one instruction and/or the at least one question. The visual information can preferably include at least one picture and/or a video, which can be output on the display of the in-home apparatus. The visual, and/or textual and/or acoustical information are developed and designed in particular for patients suffering from depressive disorder.

In order to further improve the interaction and/or conversation between the patient and the remote patient monitoring system and the in-home apparatus, respectively, the processing unit of the in-home apparatus can be preferably configured to cause the in-home apparatus
- to collect second patient related data including communication data representing answer information input by the patient in response to the treatment related information received from the first external computing system and/or current image data captured by the camera and/or face data and/or vital sign data provided by the at least one vital sign sensor device, and
- to transmit the second patient related data to the first external computing system, wherein
the processing unit of the first external computing system is configured to analyze the second patient related data received from the in-home apparatus by executing the AI-based algorithm to re-determine the patient's mental status.

Preferably, the processing unit of the first external computing system is configured to cause the first external computing system to provide in dependence of the re-determined patient's mental status further treatment related information destined for the patient.

Preferably, the remote patient monitoring system can comprise an external database system, the external database system and the first external computing system being configured to communicate with each other via the telecommunication network, wherein
the process unit of the external computing system can be configured to cause the first external computing system
- to store a patient's history list including in particular the patient's face data and/or the patient's vital sign data and/or the patient's answer information and/or the treatment related information provided by the first external computing system and/or the determined patient's mental status,
- to encrypt the patient's history list and to transmit the encrypted patient's history list to the external database system, and
- to retrieve the patient's history list from the external database system.

Preferably, the processing unit of the first external computing system can be configured to cause the first external computing system
- to execute an authentication procedure between the in-home apparatus and the first external computing system, and/or
- to decrypt encrypted patient related data received from the in-home apparatus and to encrypt patient related data destined for the in-home apparatus, and/or wherein the processing unit of the in-home apparatus can be configured to cause the in-home apparatus to initiate a communication session via the telecommunication network to the first external computing system in response to a predetermined event.

A predetermined event can be the detection of an authorized patient by processing face data captured by the camera. In this case, a software including a face recognition program can be stored in the in-home apparatus. Therefore, the processing unit can be configured to execute the face recognition program using face data captured by the camera and to cause the in-home apparatus to initiate a communication session via the telecommunication network to the first external computing system, if an authorized patient is detected.

### Brief Description of the Drawings

In the following, a preferred embodiment of the present invention is illustrated in more detail in connection with the accompanying drawings, wherein the drawings show in:
- Figure 1: a schematically block diagram of an exemplary remote patient monitoring system,
- Figure 2: a block diagram of the in-home apparatus shown in figure 1, and
- Figure 3: a block diagram of the external computing system of an mental-health-care service provider shown in figure 1.

### Detailed Description of the Invention

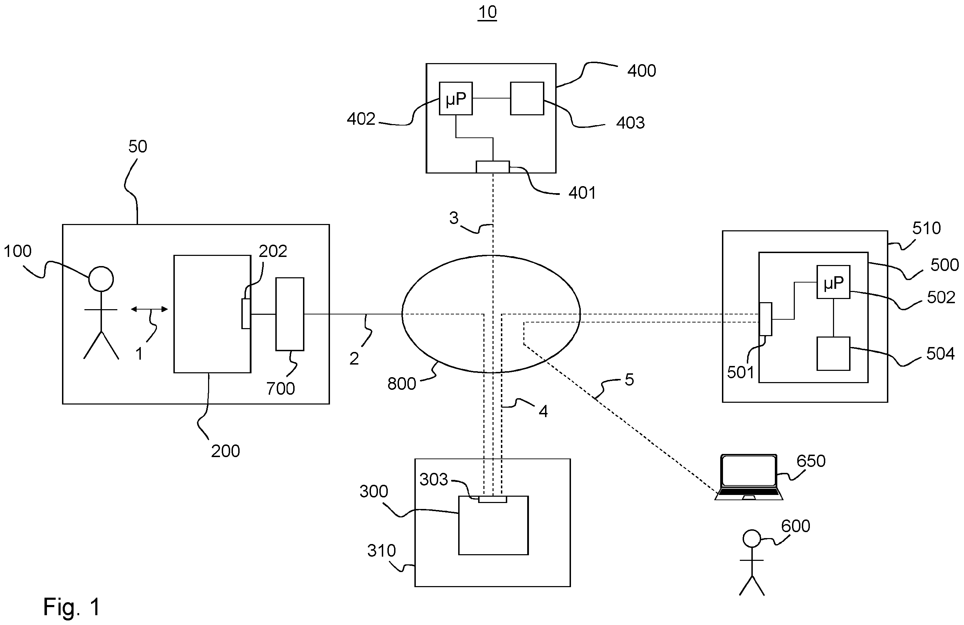

Figure 1 depicts an exemplary remote patient monitoring system 10 that is in particular configured to monitor the mental status of a patient 100 and in particular to monitor a patient 100 suffering from a depressive disorder and to provide treatment information to the patient 100.

The remote patient monitoring system 10 comprises in particular a first external computing system 300 preferably operated by a mental-health-care service provider 310, a telecommunication network 800 and at least one in-home apparatus 200. The in-home apparatus 200 may be considered as a mirror like smart apparatus, which is located at a patient's home 50. Preferably, the in-home apparatus 200 can be installed in a bathroom of the patient's home 50. The in-home apparatus 200 is configured to exchange data with the first external computing system 300 over the telecommunication network 800. The telecommunication network 800 is preferably a kind of wide area network, which may comprise the Internet and in dependence of the implementation at least one wireless or fixed wired access network.

Furthermore, the remote patient monitoring system 10 can but not have to comprise a second external computing system 400, which is preferably provided and operated by a generative AI provider. The external computing system 400 comprises a communication interface 401 configured to communicate via the telecommunication network 800 with the external computing system 300, at least one processing unit 402, e.g. a microprocessor or a microcontroller for controlling and monitoring the operation of the external computing system 400 and a storage device 403 configured to store in particular an operating system and/or a firmware and a generative AI-based algorithm. In particular, if implemented, the second external computing system 400 and the processing unit, respectively, is configured, to execute the generative AI-based algorithm to generate for example human-like conversation sentences. For example, the generative AI-based algorithm can use a Large Language Model (LLM) like a generative pre-trained transformer (GPT) used for example in ChatGPT. The first external computing system 300 and the second external computing system 400 are each configured to communicate via the telecommunication network 800.

In addition or instead of using the second external computing system 400 such a generative AI-based algorithm can be stored in and executed by the first external computing system 300.

In addition, the remote patient monitoring system 10 may further comprise an external database system 500, which is preferably provided and operated for example by a government-sanctioned medical authority. The first external computing system 300 and the external database system 500 are configured to communicate with each other via the telecommunication network 800. It is to be noted, that the external database system 500 may be considered as a cloud-based database system. The external database system 500 preferably comprises at least one processing unit 502, which can be implemented as a microprocessor or a microcontroller for controlling and monitoring the operation of the external database system 500. Further, it may comprise at least one storage device 504 and a communication interface 501. Preferably, the communication interface 501 comprises hardware and software component and implements preferably the Internet protocol (IP). The external database system 500 is configured to be connected over the telecommunication network 800 for example with a computing device 650, e.g. a note book, of a human doctor 600, e.g. psychiatrist of the patient 100 in order to may communicate relevant data concerning the mental health of a patient, e.g. the patient 100.

In figure 1, the communication between the patient 100 and the in-home-apparatus 200 is labeled 1, the communication between the in-home apparatus 200 and the external computing system is labeled 2, the communication between the external computing system 300 and the external computing system 400 is labeled 3, the communication between the external computing system 300 and the external database system 500 is labeled 5 and the communication between the external database system 500 and the computing device 650 of the psychiatrist 600 is labeled 5. It should be noted that the respective communications 2 to 5 can be conducted connection-oriented by using established communication links or connectionless.

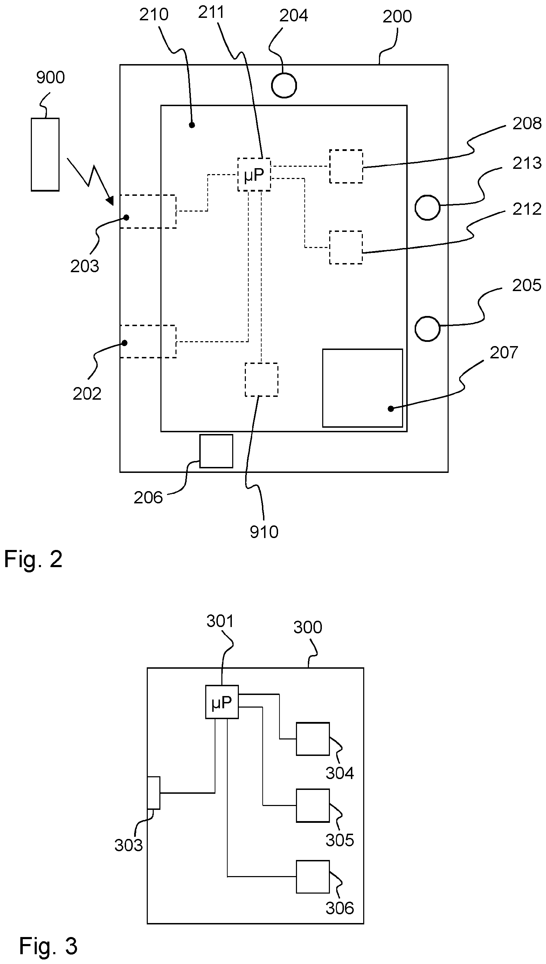

Now referring to figure 2 there is depicted in more detailed an exemplary block diagram of the in-home apparatus 200 shown in figure 1.

Preferred tasks of the in-home apparatus 200 is to collect patient related data concerning in particular the patient's mental health, to transmit the collected patient related data to the first external computing system 300, to enable a communication between the patient 100 and the in-home apparatus 200 and to output treatment related information to the patient 100.

As already mentioned the in-home apparatus 200 may be considered as a mirror like smart device, which can be installed for example in the patient's bathroom for example on a wall of the bathroom. The in-home apparatus 200 may comprise a frame or housing having a front side, a rear side, a bottom side and a top side. The in-home apparatus 200 comprises a device 210 having a light reflecting area configured to show a mirrored image of the patient 100. In an exemplary implementation, the device 210 can be a conventional mirror having, e.g. a planar surface, which is arranged on the front side of the apparatus 200. Furthermore, the in-home apparatus 200 comprises a camera 204 configured to capture image data of the patient 100, in particular if the patient 100 is standing in front of the mirror 210. Preferably, the camera 204 is positioned on the front side of the in-home apparatus and to a side, e.g. the top side of the mirror 210 as depicted in figure 2. It should be mentioned that the camera 204 can be configured to capture image data from still images and/or moving images.

Furthermore, the in-home apparatus 200 comprises a first communication interface 202, which is configured to connect the apparatus 200 directly or indirectly to the telecommunication network 800 for communicating with first the external computing system 300, which in turn is connectable to the telecommunication network 800.

Preferably, the first communication interface 202 is a network interface, implementing a standardized Wi-Fi or a standardized Ethernet communication protocol, so that the in-home apparatus 200 can be connected to an in-home router 700 as depicted in figure 1. In this case, the in-home apparatus 200 is connected via the in-home router 700 to the telecommunication network 800.

Furthermore, in-home apparatus 200 comprises a display 207 having a touch screen functionality, at least one microphone 205 and at least one speaker 206. Preferably, the display 207 can be integrated in the mirror-like device 210 as shown in figure 2. For example, the display 207 is a liquid crystal display (LCD). In particular, the display 207 can be positioned in a corner of the mirror 210 without disturbing the mirrored face of the patient 100. In particular, the display 207 is configured to output textual or visual treatment related information for the patient 100 and to be operated as an input device for the patient 100.

In an exemplary and more sophisticated implementation, the device 210 having an optical reflecting area and the display 207 can be implemented integrally such that the optical reflecting area is configured to operate as a mirror and/or a display, wherein the optical reflecting area has a touch screen functionality. In other words: The device 210 may be configured to be operated as a mirror and a display touchscreen. Such a device may be called herein a touch-enabled mirror-display device.

In the exemplary embodiment the camera 204, the display 207, the microphone 205 and the speaker 206 are arranged relatively to the mirror 210 such that they have an optimized position when the patient 100 is standing directly in front of the mirror 210. Preferably, the at least one microphone 205 and the at least one speaker 206 are positioned to a side of the device 210 as depicted in figure 2.

In addition, the in-home apparatus 200 includes at least one processing unit 211, which preferably is implemented as a microprocessor or microcontroller. The processing unit 211 is connected to the display 207, the at least one microphone 205, the at least one speaker 206 and the communication interface 202 and, if available, to the device 210 implemented as a touch-enabled mirror-display device. A first storage device 208 can be implemented in the in-home apparatus 200, which is configured to store software for example an operating system or a firmware for controlling and monitoring the operation of the in-home apparatus 200. The processing unit 211 is connected to the first storage device 208 and configured to execute the software stored in the storage device 208. For example, the processing unit 211 and the storage device 208 can be components of a microcontroller.

Furthermore, at least one vital sign sensor device is communicatively coupled to the in-home apparatus 200. For example, at least one vital sign sensor device 910 connected to the processing unit 211 can be implemented in the in-home apparatus 200. In addition or alternatively at least one external virtual sign sensor device 900 can be communicatively coupled with the in-home apparatus 200 and the processing unit, respectively, via at least one second communication interface 203 implemented in the in-home apparatus 200. Preferably, the external vital sign sensor device 900 is a wearable sensor device, e.g. a smart watch, or an implantable sensor device carried by the patient 100. In this case, the second communication interface 203 is implemented as a wireless network interface, which preferably implements a wireless short-range communication protocol such as Bluetooth. As a result, the external vital sign sensor device 900 is configured to communicate with the in-home apparatus 200 using the respective wireless short-range communication protocol. According to an exemplary implementation, the external vital sign sensor device 900 is configured to sense vital signs of the patient 100 and to generate vital sign data, which represents for example the blood pressure of the patient 100. The internal vital sign sensor device 910 is configured to detect vital signs of the patient 100 and to generate vital sign data, which, for example, represents the temperature of the patient 100.

The processing unit 211 is configured to cause, when executing the operating system stored in the storage device 207, the in-home apparatus 200 to collect patient related data including at least one of
- image data captured by the camera 204,
- vital sign data provided by the vital sign sensor detector 900 and/or the vital sign sensor device 910 and
- communication data representing information input by the patient via the display 207 and/or the microphone 205 preferably in response to treatment related information output via the speaker 206 and/or the display 207 to the patient 100. The treatment related information includes at least one instruction and/or at least one question for the patient 100 and, if applicable multimedia data, wherein the at least one instruction can be an initial instruction stored in the in-home apparatus 200 and/or wherein the least one question can be an initial question also stored in the in-home apparatus 200. Treatment related information destined for the patient 100 can also be transmitted by the external computing system 300 to the in-home apparatus 200. Furthermore, the processing unit 211 is configured to cause the in-home apparatus 200 to deliver the collected patient related data to the first communication interface 202 for transmitting them to the external computing system 300.

It is to be noted, that the processing unit 211 can be configured to store the collected patient related data in the storage device 208 or in separate storage device 212.

In order to support a voice communication between the patient 100 and the in-home apparatus 200 a speech-to-text conversation program and/or a text-to speech conversation program can be stored for example in the storage device 208. Therefore, the processing unit 211 is preferably configured to cause the in-home apparatus 200 to execute a speech to-text conversation on speech signals input by the patient 100 via the microphone 205 and preferably destined for the external computing system 300, and/or to execute a text-to-speech conversation on textual data received from the external computing system 312 and/or stored in the in-home apparatus 200 and to output the corresponding speech signals via the speaker 206.

In addition, a program controlling an authentication procedure between the in-home apparatus 200 and the external computing system 300 and/or a program for decrypting and encrypting data can be stored for example in the storage device 208.

Therefore, the processing unit 211 can be configured to cause the in-home apparatus 200 to control and/or initiate an authentication procedure between the in-home apparatus 200 and the external computing system 300 by executing the respective program and/or to decrypt encrypted data received from the external computing system 300 and to encrypt patient related data destined for the external computing system 300 and/or to grant only an authorized patient access to the in-home apparatus 200. For example, in order to get access to the in-home apparatus 200 a fingerprint sensor (not shown) can be used by the patient 100 or the patient 100 can input a password on the in-home apparatus 200. Preferably, an authorized patient can be detected by processing face data captured by the camera 204. In this case, a software including a face recognition program can be stored for example in the storage device 208 of the in-home apparatus 200. Therefore, the processing unit 211 can be configured to execute the face recognition program using face data captured by the camera 204 and to cause the in-home apparatus 200 to grant access if an authorized patient has been detected. Other mechanism granting access are well known.

In order to increase the performance of in-home apparatus 200 a facial expression recognition program can be stored for example in the storage device 208. In this case, the processing unit 211 is configured to obtain, when executing the facial expression recognition program faced data from the image data captured by the camera 204 and to cause the in-home apparatus 200 to deliver the face data alone or together with the image data to the first communication interface 200 for transmitting them to the external computing system 300. The face data represents facial expressions of the patient 100.

It is to be noted that the in-home apparatus 200 is preferably configured to protect the patients 100 by the camera 204 and/or the microphone 206.

Preferably, the processing unit 211 of the in-home apparatus 200 can be configured to cause the in-home apparatus 200 to initiate a communication session via the telecommunication network 800 to the first external computing system 300 in response to a predetermined event. Therefore, the in-home apparatus 200 knows an address information, e.g. the IP-address of the external computing system 300 to initiate the establishment of a communication session via the telecommunication network 800 to the external computing system 300. The predetermined event can be a time-based trigger provided by a timer (not shown) of the in-home apparatus 200 or the detection of a movement of the patient 100 for example by a motion sensor 213 of the in-home apparatus 200. Such a predetermined event may also be detected by the in-home apparatus 200 for example if the vital signs detected by the vital sign sensor device 900 and/or 910 indicates an emergent mental state of patient 100.

Alternatively, such a predetermined event can be the detection of an authorized patient. Preferably, an authorized patient can be detected by processing face data captured by the camera 204. In this case, a software including a face recognition program can be stored for example in the storage device 208 of the in-home apparatus 200. Therefore, the processing unit 211 can be configured to execute the face recognition program using face data captured by the camera 204 and to cause the in-home apparatus 200 to initiate a communication session to the external computing system 300, if an authorized patient has been detected.

Referring now to figure 3 there is depicted an exemplary block diagram of the external computing system 300 shown in figure 1.

The external computing system 300 can be also considered as a cloud based computing system. The external computing system 300 and the in-home apparatus 200 are configured to communicate via the telecommunication network 800 with each other.

To that end the external computing system 300 comprises at least one communication interface 303 configured to connect the external computing system 300 to the telecommunication network 800. Preferably, the communication interface 303 comprises hardware and/or software components and implements for example the Internet protocol (IP).

It is to be noted that in dependence of the implementation face data representing facial expressions of the patient 100 can be generated a) by the in-home apparatus 200 or b) by the external computing system 300 itself.

In case a), the external computing system 300 can be configured to receive via its communication interface 303 first patient related data including vital sign data of the patient, and/or communication data and/or face data representing facial expressions of the patient 100 transmitted by the in-home apparatus 200 via the telecommunication network 800.

Furthermore, the external computing system 300 comprises
- a storage device 304 configured to store software, which comprises in particular an operating system, and/or a firmware for controlling and monitoring the operation of the external computing system 300 and an AI-based algorithm for determining the patient's mental status, and
- a processing unit 301 configured
to analyze the first patient related data received from the in-home apparatus 200 by executing an AI-based algorithm to determine the patient's mental status, to provide in dependence of the determined patient's mental status treatment related information including at least one instruction and/or question for the patient and to transmit the treatment related information to the in-home apparatus, wherein the in-home apparatus is configured to output the treatment related information received from the first external computing system 300 via the display 207 and/or the speaker 206.

It is to be noted that the mental status of a patient, e.g. patient 100, in particular deflects the mental status for a patient suffering from depressive disorder.

It is noted that the AI-based algorithm uses in particular a machine learning algorithm, wherein preferably vital sign data of the patient and/or face data representing facial expressions of the patient and/or communication data input by the patient via the microphone and/or the display can be use an input data for the AI-based algorithm, wherein its output defines a mental status of the patient 100.

In case b), the external computing system 300 can be configured to receive via its communication interface 303 patient related data including vital sign data of the patient, and/or communication data and/or image data transmitted by the in-home apparatus 200 via the telecommunication network 800. It is to be noted that in this case the storage device 304 is configured to store in particular an operating system, and/or a firmware for controlling and monitoring the operation of the external computing system 300, an AI-based algorithm for determining the patient's mental status and a facial expression recognition program. In order to be able to process image data received from the in-home apparatus 200 the processing unit 301 is configured to cause the first external computing system 300
- to obtain when executing the facial expression recognition program face data from the image data received from the in-home apparatus 200, the face data representing facial expressions of the patient 100,
- to analyze the first patient related data including the obtained face data and the vital sign data received from the in-home apparatus 200 by executing the AI-based algorithm to determine the patient's mental status,
to provide in dependence of the determined patient's mental status a treatment related information including at least one instruction and/or question for the patient and to transmit the treatment related information to the in-home apparatus 200, wherein the in-home apparatus 200 is configured to output the treatment related information received from the first external computing system 300 via the display 207 and/or the speaker 206. It is to be noted, that the treatment related information provided by the first external computing system 300 may further includes multimedia information such as visual, and/or textual and/or acoustical information preferably associated with the at least one provided instruction and/or question.

The step of providing a treatment related information for a patient, e.g. patient 100, in dependence of the determined patient's mental status can be executed by generating such a treatment related information. Therefore, in order to be able to provide a treatment related information for the patient 100, the external computing system 300 can preferably comprise a storage device 306, e.g. a treatment database, which may store, a plurality of treatment elements, e.g. predetermined high-level actions like a plurality of pictures, a plurality of videos, pieces of music and/or sports exercises, a list of predetermined questions and/or predetermined instructions. An exemplary instruction can be "Select two pictures", "Read the text", "Watch a sport video and do the shown exercises", "Listen to the music" etc.

In this case the processing unit 301 is preferably configured to cause the first external computing system 300 to generate in dependence of a determined patient's mental status, and, if applicable, in dependence of a previously determined and stored patient's mental status and previously provided and stored treatment related information for the patient 100 a current treatment related information for the patient 100. This can be achieved in particular by selecting some of the treatment elements stored in the storage device 306 in particular on the basis of a predefined policy.

In practice, the instruction and/or question of a treatment related information destined for a patient, e.g. the patient 100, is impersonal. Therefore, the processing unit 301 can be configured to cause the external computing system 300 to generate in dependence of the instruction and/or question of the treatment related information a prompt message that requests the second external computing system 400 to generate a human-like conversation sentence and to transmit the prompt message to the second external computing system 400. Therefore, the external computing system 300 knows an address, e.g. the IP-address of the external computing system 400 to send the prompt message via the telecommunication network 800 to the external computing system 400. The second external computing system 400 is configured to execute the generative AI-based algorithm stored in the storage device 403 to generate in response to the received prompt message a corresponding human-like conversation sentence and to transmit the human-like conversation sentence to the first external computing system 300.

Instead of using the separate external computing system 400, a generative AI-based algorithm as illustrated above can be stored for example in the storage device 304 of the external computing device 300. In this case, the processing unit 301 can be configured to cause the external computing system 300
- to execute a generative AI-based algorithm to generate a human-like conversation sentence in dependence of the at least one predetermined instruction and/or the at least one predetermined question provided by the first external computing system,
- to transmit the generated human-like conversation sentence to the in-home-apparatus 200, wherein the processing unit 211 of the in-home apparatus 200 is configured to cause the in-home-apparatus 200 to output the human-like conversation sentence via the display 207 and/or the speaker 206.

In a preferred embodiment, the processing unit 301 is configured to cause the external computing system 300 to keep track of all information concerning a patient, e.g. patient 100, i.e.
- the patient related information collected by the in-home apparatus 200 and transmitted to the external computing system 300 and
- the treatment related information provided with respect to patient 100 and
to store these information as patient related history list for example in a storage device 305. Preferably, all these information concerning a patient are stored in the storage 305 during an ongoing communication session between the in-home apparatus 200 and the patient 100, respectively and the external computing system 300. Preferably at the end of a communication session the history list stored in storage 305 is transmitted from the external computing system 300 to the external database system 500 and then deleted from the storage device 305. Preferably, all information concerning a patient, e.g. patient 100, is encrypted before it is stored and transmitted to the external database system 500. Once the history list is needed the external computing system 300 is configured to retrieve the history list from the external database system 500.

In an exemplary implementation, predetermined questions or instructions concerning patient 100 may by input by the psychiatrist 600 into its notebook 650 and then transmitted to the external database 500 which in turn is configured to transmit in a push or a pull mode the received predetermined questions or instructions concerning patient 100 to the external communication system 300. It is to be noted, that the remote patient monitoring system 10 and in particular the external computing systems 300, 400, the in-home apparatus 200, the external database system 500 and the computing device 650 are each configured to ensure privacy protection of a patient, e.g. patient 100.

In addition, the software stored for example in the storage device 304 may further comprise an authentication program configured to control, if executed by the processing unit 301, an authentication procedure between the external computing system 300 and the in-home apparatus 200 and the patient 100, respectively, and/or a program for encrypting or decrypting data, and/or a privacy protection program.

Next, the operation of the remote patient monitoring system 10 as shown in figure 1 is illustrated in more detail with respect to an exemplary communication session.

It is assumed, that a unique IP-address is temporarily or permanently assigned to each of the in-home apparatus 200, the external computing systems 300 and 400, the external database system 500 and the notebook 650. Furthermore, it is assumed that a plurality of treatment elements are stored for example in the storage device 306 which may comprise at least one predetermined instruction and/or at least one predetermined question and/or multimedia content, e.g. textual, and/or acoustical and/or visual information concerning a patient, which suffers from a mental disorder in particular from a depressive disorder. An exemplary treatment element is the instruction "Select two pictures from the group of pictures output on the display", wherein another exemplary treatment element includes a group of predetermined pictures, e.g. 8 different pictures.

It is to be noted, that instructions and questions concerning patient 100 can be entered by the mental health doctor 600 on its notebook 650, transmitted via the telecommunication network 800 to the external database system 500 and retrieved by the external computing system 300 when necessary.

Moreover, it is assumed that data identifying patient 100 are stored in the external computing system 300 for example in storage device 306 and that patient 100 is registered on the external computing system 300 and the mental-health-care service provider 310, respectively.

It is now assumed, that the in-home apparatus 200 has detected a motion of patient 100. In response to the detected motion the processing unit 211 causes the in the in-home apparatus 200 to establish a communication session to the external computing system 300 and to start an authentication procedure between the in-home apparatus 200 and the external communicating system 300.

In addition, the processing unit 211 triggers the camera 204 to scan the patient 100 that is standing in front of the mirror 210 to capture image data. In addition, the process unit 211 further triggers the vital sign sensor device 900 and 910 to detect vital signs concerning for example the blood pressure and the body temperature, respectively and to generate respective vital sign data. In particular the image data generated by the camera 204 and the vital sign data generated by the vital sign sensor device 900 and 910 are collected under control of the processing unit 211 and stored for example in the storage device 212.

In the present case, it is assumed that a facial expression recognition program is stored in the storage device 208 of the in-home apparatus 200. Therefore the processing unit 211 now executes the facial expression recognition program to obtain face data from the image data of the camera 204, wherein the face data representing facial expressions of the patient 100. The face data are also stored in the storage device 212. According to a preferred implementation, a current detecting timestamp is associated with the detected vital sign data and the detected image data and the face data, respectively, is also stored in storage device 212.

It is further assumed, that at least one predetermined initial question, e.g. "How are you feeling right now?", and/or at least one initial instruction, e.g. "Do 10 push-ups", are stored for example in the storage device 208. The processing unit 211 is configured to output for example now the initial question via the display 207 or after performing a text-two speech-conversion via the speaker 206. In response to the initial question the patient 100 inputs a answer, e.g. "I am feeling extremely anxious" via the display 207 or via the microphone 205, wherein the respective answer data can be stored for example in storage in device 212 together with the other patient related data including the image data and the face data, respectively, and vital sign data.

Now it is presumed, that the authentication procedure between the in-home apparatus 200 and the external computing system 300 has been successfully finished. As a result, the processing unit 301 of the external computing system 300 establishes a communication link via the telecommunication network 800 to the external data base system 500 and transmits a requesting message to the database system 500 requesting for transmitting, if available, the history list of patient 100 and/or questions and/or instructions with respect to patient 100 to the external computing system 300.

Now the processing unit 211 causes the in-home apparatus 200 to transmit all collected patient related data stored in the storage device 212 via the telecommunication network 800 to the external computing system 300. The collected patient related data comprises the answer data input by the patient 100, the face data and the vital sign data and, if available the current timestamp. It is to be noted, that prior to transmitting the patient related data, the processing unit 211 is configured to encrypt the collected patient related data and to transmit the encrypted patient related data to the external computing system 300.

The processing unit 301 is configured to cause the external computing system 300 to store the patient related data received from the in-home apparatus 200 for example in storage device 305. Furthermore, the processing unit 301 is configured to decrypt the received patient related data and to analyzes the patient related data, in particular the face data, the answer data and the vital sign data received from the in-home apparatus 200 by executing the AI-based algorithm to determine the patient's mental status. Thereafter, the processing unit 301 causes the external computing system 300 to generate by executing the action generating program in dependence of the determined patient's mental status at least one treatment related information, which includes for example at least one predetermined instruction and/or at least one predetermined question and/or predetermined multimedia data for patient 100. In the present case, the processing unit 301 generates a treatment related information, which includes the instruction "Select two pictures from the group of pictures output on the display" and a further treatment element including the group of 8 different pictures.

Since the instruction "Select two pictures from the group of pictures output on the display" is very impersonal it might be desirable to generate a more personal human-like conversation sentence. Therefore, the processing unit 301 can be configured to cause the external computing system 300 to generate a prompt message, e.g. the prompt message:
"Act as a professional psychiatrist. Provide a professional advice to a patient. I want the patient do the following action: 'Select two pictures from the group of pictures output on the display'. Remember that your tone must sound personal and give a clear motivation so that the patient will likely do the action. This is a conversation between the patient and psychiatrist. Be brief and provide it as a sentence. Pretend you are in a therapy session with the patient. Patient's name is John."

It is to be noted that a plurality of prompt templates can be stored for example in the storage device 304, which can be used by the processing unit 301. Furthermore, it is to be noted that in order to protect the patient's identity John is not the real name of the patient 100. The generated prompt message is transmitted from the external computing system 300 via the telecommunication network 800 to the external computing system 400. The processing unit 402 is configured to execute the generative AI-based algorithm stored in the storage device 304 to generate a human-like conversation sentence with respect to received prompt message. For example, the generative AI-based algorithm generates the following human-like conversation sentence: "John, I see you're looking at the screen. Would you please select two pictures that speak to you the most? Engaging with these images can help us better understand your feelings and thoughts. It's a simple yet powerful way to explore your emotional landscape."

Preferably, the processing unit 402 causes the external computing system 400 to encrypt the generated human-like conversation sentence and transmit the encrypted sentence the external computing system 300.

The processing unit 301 is configured to cause the external computing system 300
- to replace in the human-like conversation sentence the name "John" by the real name of the patient 100, which known to the external computing system 300, and
- to transmit, using the IP-address of the in-home apparatus 200, the complete treatment related information including in encrypted form as an instruction the human-like conversation sentence and the group of 8 different pictures via the telecommunication network 800 to the in-home apparatus 200. In addition the processing unit 301 is configured to store this treatment related information together with the detected mental status of the patient 100 in a history list associated with the patient 100 for example in storage device 305 in an encrypted form.

The processing unit 211 is configured to cause the in-home apparatus 200
- to decrypt the received treatment related information including the human-like conversation sentence and the group of 8 pictures and
- for example to execute a text-to-speech conversation on the human-like conversation sentence, and
to output the converted human-like conversation sentence via the speaker 206 and to output the group of 8 pictures on the display 207.

As instructed, the patient 100 now selects two pictures from the group of 8 pictures for example by touching the display 207 on the corresponding places. The processing unit 211 is further configured to cause the in-home apparatus 200 to collect the answer information corresponding to the two selected pictures and store the answer for example in the storage device 212.

Next, if applicable, the processing unit 211 can be configured to trigger again the camera 204 to scan the patient 100 that is still standing in front of the mirror 210 to capture second image data. In addition, the process unit 211 further triggers the vital sign sensor device 900 and 910 to detect second vital signs concerning for example the blood pressure and the body temperature, respectively and to generate respective vital sign data. In particular the second image data generated by the camera 204 and the second vital sign data generated by the vital sign sensor device 900 and 910 are collected under control of the processing unit 211 and stored for example in the storage device 212 together, if available, with a second detecting time stamp.

Next, the processing unit 211 now executes the facial expression recognition program to obtain second face data from the second image data of the camera 204, wherein the face data representing facial expressions of the patient 100. The second face data are also stored in the storage device 212.

Now the processing unit 211 causes the in-home apparatus 200 to transmit all collected second patient related data stored in the storage device 212 via the telecommunication network 800 to the external computing system 300. The collected second patient related data comprises the answer data input by the patient 100 via the display 207, the second face data and the second vital sign data and, if available the second timestamp. It is to be noted, that prior to transmitting the patient related data, the processing unit 211 is configured to encrypt the collected patient related data and to transmit the encrypted second patient related data to the external computing system 300.

The processing unit 301 is configured to cause the external computing system 300 to store the second patient related data received from the in-home apparatus 200 for example in storage device 305. Furthermore, the processing unit 301 is configured to decrypt the received second patient related data and to analyze the second patient related data, in particular the second face data, the answer data and the second vital sign data received from the in-home apparatus 200 by executing the AI-based algorithm to re-determine the patient's mental status. The re-determent patient's mental status can be stored together with the second patient related data received from the in-home apparatus 200 for example in the history list stored in the storage device 305 preferably in an encrypted from.

In dependence of the implementation, the processing unit 301 may be configured to cause the external computing system 300
- either to generate by executing the action generating program in dependence of the re-determined patient's mental status a further treatment related information and to continue the process as illustrated above with respect to the first patient related data or
- to transmit in an encrypted form the history list of the patient 100 via the telecommunication network 800 to the external database system 500, wherein the history list is then stored in the storage device 504, and
- to terminate the established communication session between the external computing system 300 and the in-home apparatus 200.

According to a preferred implementation, the termination of the communication session can be signaled to the patient 100 via an acoustical information output by the speaker 206 and/or a visual information output by the display 207.

The processing unit 502 of the external database system 500 is preferably configured to cause the external database system 500 to store the received history list, which may include all patient related data and treatment related information generated during the communication session between the in-home apparatus 200 and the external computing system 300.

The processing unit of 502 is preferably configured to cause the external database system 500 to transmit the stored conversation history list using a push or pull mode to the notebook 650 of the psychiatrist 600. The computing device 650 can be configured to decrypt the received encrypted history list of the patient 100. In dependence of the history list the psychiatrist 600 can input new instructions and/or questions and/or other feedback information on the notebook 650. The notebook 650 can be configured to encrypt the instructions and/or questions and/or other feedback information and to transmit them to the external database system 500.

In particular, if a new communication session is initiated by the in-home apparatus 200 with the external computing system 300, the external computing system 300 is configured to retrieve the history list of the patient 100 as well as the instructions and/or questions and/or other feedback information input by the psychiatrist 600 from the external database system 500. It is to be noted, that a new treatment related information can be provided to the patient 100 considering the retrieved history list of the patient 100 as well as the instructions and/or questions and/or other feedback information input by the psychiatrist 600.

In particular, the external computing system 300 is configured to send a respective request message to the external database system 500 requesting for transmission of the history list of the patient 100 as well as the instructions and/or questions and/or other feedback information input by the psychiatrist 600.

It should be mentioned, that if considered useful or necessary the psychiatrist 600 can decide to initiate an in-person consultation with the patient 100 in dependence of the latest received history list.

Is to be noted that according to a preferred implementation the processing unit 211 can be configured to cause the in-home apparatus 200 to periodically monitor the patient 100. This can be achieved in that the in-home apparatus 200 periodically collects vital sign data from the vital signs sensor device 900 and/or 910 and/or image data from the camera 204. The periodically collected data can be stored for example in the storage device 212 and transmitted later on to the external computing system 300, once a communication session is established.

Once the in-home apparatus 200 is re-triggered by a predetermined event the exemplary sequence of communication session as illustrated above in connection with the figures 4a and 4b is performed by the remote patient monitoring system 10 again.

## Claims

1. An in-home apparatus (200) in particular for use in a remote patient monitoring system, the apparatus (200) comprising:
a device (210) having a light reflecting area configured to show a mirrored image of a patient (100),
a camera (204) configured to capture image data of a patient (100),
a first communication interface (202) configured to connect the in-home apparatus (200) to a telecommunication network (800) for communicating with an external computing system (300) connectable to the telecommunication network (800),
a display (207) having a touch screen functionality, a microphone (205) and a speaker(206,
a processing unit (211) configured
a) to cause the in-home apparatus (200) to collect patient related data including image data captured by the camera (204), and/or vital sign data provided by at least one vital sign sensor device (900, 910) configured to detect vital signs of the patient and to generate vital sign data, and/or communication data input by the patient via the display (207) and/or the microphone (205), and
b) to cause the in-home apparatus (200) to deliver the collected patient related data to the first communication interface (202) for transmitting them to the external computing system (300).

2. The in-home apparatus of claim 1, wherein
the at least one vital sign sensor device (910) is an integral sensor device of the in-home apparatus.

3. The in-home apparatus of claim 1, wherein
the at least one vital sign sensor device (900) is an external sensor device and wherein the in-home apparatus (200) further comprises at least one second communication interface (203) configured to be communicatively coupled with the external vital sign sensor device (900).

4. The in-home apparatus of claim 3, wherein
the at least one second communication interface (203) is implemented according to a short-range wireless communication protocol and wherein the at least one external sensor device (900) is a wearable sensor device or an implantable sensor device.

5. The in-home apparatus of any one of the claims, wherein
the vital sign data represents the body temperature, the blood pressure, the heart rate or the respirating rate of the patient.

6. The in-home apparatus of claim 5, wherein
the processing unit (211) is configured to cause the in-home apparatus (200)
- to execute a speech-to-text conversion on speech signals input by the patient via the microphone (205) and/or
- to execute a text-to-speech conversion on data representing text received from the external computing system (300) and to output the corresponding speech signals via the speaker (206).

7. The in-home apparatus of any one of the preceding claims, wherein
the processing unit (211) is configured to cause the in-home apparatus (200)
- to execute an authentication procedure between the in-home apparatus (200 and the external computing system (300), and/or
- to decrypt encrypted data received from the external computing system (300) and to encrypt patient related data destined for the external computing system (300), and/or
- to grant only an authorized patient access to the in-home apparatus (200).

8. The in-home apparatus of any one of the preceding claims comprising
a storage device (208) configured to store software including a facial expression recognition program, wherein
the processing unit (211) is configured
to obtain when executing the facial expression recognition program face data from the image data, the face data representing facial expressions of the patient (100), and
to cause the in-home apparatus (100) to deliver the face data to the first communication interface (202) for transmitting them to the external computing system (300).

9. A remote patient monitoring system (10) in particular for monitoring a patient's mental status, comprising:
an in-home apparatus (200) of claim 8, the in-home apparatus (200) located at a patient's home (50),
a telecommunication network (800),
a first external computing system (300), the first external computing system (300) and the in-home apparatus (200) being configured to communicate with each other via the telecommunication network (800), wherein
the first external computing system (300) is configured to receive from the in-home apparatus (200) first patient related data including vital sign data of the patient, and/or communication data input by the patient via the microphone (205) and/or the display (207) and/or face data representing facial expressions of the patient, wherein the first external computing system (300) comprises:
- a communication interface (303) configured to connect the external computing system (300) to the telecommunication network (800),
- a storage device (304) configured to store an AI-based algorithm for determining the patient's mental status, and
- a processing unit (301) configured
to analyze the first patient related data received from the in-home apparatus (200) by executing an AI-based algorithm to determine the patient's mental status,
to provide in dependence of the determined patient's mental status treatment related information including at least one instruction and/or question for the patient and
to transmit the treatment related information to the in-home apparatus (200),
wherein the in-home apparatus (200) is configured to output the treatment related information received from the first external computing system (300) via the display (207) and/or the speaker (206).

10. A remote patient monitoring system (10) in particular for monitoring a patient's mental status, comprising:
an in-home apparatus (200) of any one of the claims 1 to 7, the in-home apparatus (200) located at a patient's home (50),
a telecommunication network (800),
a first external computing system (300), the first external computing system (300) and the in-home apparatus (200) being configured to communicate with each other via the telecommunication network (800), wherein
the first external computing system (300) is configured to receive from the in-home apparatus (200) first patient related data including image data of the patient, and/or communication data input by the patient via the microphone (205) and/or the display (207) and/or vital sign data of the patient, wherein the first external computing system (300) comprises:
a communication interface (303) configured to connect the first external computing system (300) to the telecommunication network (800),
a storage device (304) configured to store software including a facial expression recognition program and an AI-based algorithm for determining the patient's mental status, and
a processing unit (301) configured to cause the first external computing system (300)
- to obtain when executing the facial expression recognition program face data from the image data received from the in-home apparatus (200), the face data representing facial expressions of the patient (100),
- to analyze the obtained face data and/or the vital sign data and or the communication data received from the in-home apparatus (200) by executing an AI-based algorithm to determine the patient's mental status,
to provide in dependence of the determined patient's mental status treatment related information including at least one instruction and/or question for the patient and
to transmit the treatment related information to the in-home apparatus (200),
wherein the in-home apparatus (200) is configured to output the treatment related information received from the first external computing system (300) via the display (207) and/or the speaker (206).

11. The remote patient monitoring system of claim 9 or 10, wherein either
a) the remote patient monitoring system (10) comprises a second external computing system (400) configured to execute a generative AI-based algorithm, wherein
the processing unit (301) of the first external computing system (300) is configured
- to cause the first external computing system (300) to prompt the second external computing system (400) to execute the generative AI-based algorithm to generate a human-like conversation sentence using the at least one instruction and/or the at least one question of the treatment related information provided by the first external computing system (300), wherein the second external computing system (400) is configured to transmit the human-like conversation sentence to the first external computing system (300), or wherein
b) the processing unit (301) of the first external computing system (300) is configured to execute a generative AI-based algorithm stored in the storage device (304) to generate a human-like conversation sentence with respect to the at least one predetermined instruction and/or question provided by the first external computing system (300), wherein
the processing unit (301) of the first external computing system (300) is configured to cause the first external computing system (300) to transmit the human-like conversation sentence to the in-home-apparatus (200), wherein the processing unit (211) of the in-home apparatus (200 is configured to cause the in-home-apparatus (200) to output the human-like conversation sentence via the display (207) and/or the speaker (206) .

12. The remote patient monitoring system of any one of the claims 9 to 11,
wherein
the treatment related information provided by the first external computing system (300) in dependence of the determined patient's mental status further includes visual, and/or textual and/or acoustical information.

13. The remote patient monitoring system of any one of the claims 9 to 12,
wherein
the processing unit (211) of the in-home apparatus (200) is configured to cause the in-home apparatus (200)
- to collect second patient related data including communication data representing answer information input by the patient (100) in response to the treatment related information received from the first external computing system (300) and/or image data captured by the camera (204) and/or vital sign data provided by the at least one vital sign sensor device (900, 910), and
- to transmit the second patient related data to the first external computing system (300), wherein
the processing unit (301) of the first external computing system (300) is configured to analyze the second patient related data received from the in-home apparatus (200) by executing the AI-based algorithm to re-determine the patient's mental status.

14. The remote patient monitoring system of claim 13, wherein
the processing unit (301) of the first external computing system (300) is configured to cause the first external computing system (300) to provide in dependence of the re-determined patient's mental status further treatment related information destined for the patient (100).

15. The remote patient monitoring system of any one of the claims 9 to 14, comprising
an external database system (500), the external database system (500) and the first external computing system (300) being configured to communicate with each other via the telecommunication network (800), wherein
the processing unit (301) of the first external computing system (300) is configured to cause the first external computing system (300)
- to store a patient's history list including the patient's face data and/or the patient's vital sign data and/or the patient's answer information and/or the treatment related information provided by the first external computing system (300) and/or the determined patient's mental status
- to encrypt the patient's history listand to transmit the encrypted patient's history list to the external database system (500), and
- to retrieve the encrypted patient's history list from the external database system (500).

16. The remote patient monitoring system of any one of the claims 9 to 15,
wherein
the processing unit (301) of the first external computing system (300) is configured to cause the first external computing system (300)
- to execute an authentication procedure between the in-home apparatus (200) and the first external computing system (300), and/or
- to decrypt encrypted patient related data received from the in-home apparatus (200) and to encrypt patient related data destined for the in-home apparatus (200), and/or wherein
the processing unit (211) of the in-home apparatus (200) is configured to cause the in-home apparatus (200) to initiate a communication session via the telecommunication network (800) to the first external computing system (300) in response to a detected predetermined event.
